# EUROPEAN PATENT APPLICATION

(11) **EP 2 027 847 A1**
(43) Date of publication of application: **25.02.2009**
(21) Application number: 07737236.5
(22) Date of filing: 30.05.2007
(51) Int. Cl.: A61K 8/49, A61K 8/19, A61K 8/27, A61K 8/29, A61Q 17/04

(54) **SUNSCREEN PREPARATIONS**

(30) Priority: 01.06.2006 JP 2006153859; 01.06.2006 JP 2006153860
(71) Applicant: Shiseido Company, Limited, Chuo-ku Tokyo 104-8010 (JP)
(72) Inventor: YAJIMA, Isao, Yokohama-shi, Kanagawa 224-8558 (JP); ABE, Koji, Yokohama-shi, Kanagawa 224-8558 (JP); ARAKI, Hidefumi, Yokohama-shi, Kanagawa 224-8558 (JP)
(74) Representative: Merkle, Gebhard
(86) International application number: PCT/JP2007/000580
(87) International publication number: WO 2007/138750

(57) **Abstract**

The present invention is a sunscreen cosmetic comprising a benzotriazole derivative represented by the following general formula (I) and metal oxide powder whose surface is treated with an alkylalkoxy silane.

The present invention is a sunscreen cosmetic comprising an ultraviolet absorbent having absorption in the UV-A region and metal oxide powder whose surface is treated with an alkylalkoxy silane.

The object of the present invention is to provide a sunscreen cosmetic that prevents staining due to secondary adhesion to clothing.
General formula (I)
(In this formula, R' = a straight chain alkyl group of C1-C6 and R" = a straight chain alkyl group of C1-C3.)

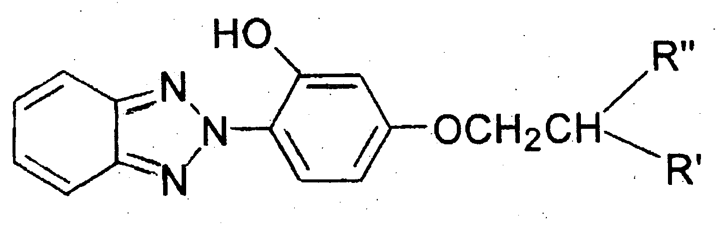

## Description

### TECHNICAL FIELD

### [First and second inventions]

The present invention relates to a sunscreen cosmetic. More specifically, it relates to a sunscreen cosmetic that prevents staining due to secondary adhesion to clothing.

### BACKGROUND ART

### [First and second inventions]

Important ultraviolet wavelength regions absorbed by sunscreen cosmetics are the UV-A region (320-400 nm) and UV-B region (290-320 nm). It was believed that the ultraviolet light in the UV-A region darkened the skin but it would not cause sunburn and accelerate aging of the skin as the ultraviolet light in the UV-B region would.

However, in recent years, it has been made clear that, whereas the ultraviolet light in the UV-B region only reaches the surface part of the skin, the ultraviolet light in the UV-A region reaches the deeper part of the skin and induces not only skin aging but also skin cancer. Therefore, there is an increasing demand for sunscreen cosmetics to have ultraviolet absorption in the UV-A region.

There are many kinds of ultraviolet absorbents that are blended into sunscreen cosmetics; 4-(1,1-dimethylethyl)-4'-methoxy dibenzoylmethane is used as an ultraviolet absorbent for the UV-A region.

In order to increase the ultraviolet shielding effect of a sunscreen cosmetic, inorganic powder ultraviolet shielding agents are sometimes added along with the ultraviolet absorbent. However, having 4-(1,1-dimethylethyl)-4'-methoxy dibenzoylmethane and inorganic powder at the same time causes the fatal problem of discoloring in the base agent. Patent Document 1 solves this problem by using a surface treatment with a silicone compound such that 1.25 x 10⁻³ mol or more silicon atoms are contained per 100 m² of the inorganic powder surface area.

Patent Document 2 describes the addition of at least one inorganic titanium oxide pigment treated with silane and/or silicone in an attempt to prevent yellow discoloration of a dibenzoylmethane derivative selected from a group consisting of 2-methylbenzoylmethane, 4-methyldibenzoylmethane, 4-isopropyldibenzoylmethane, 4-tert-butyldibenzoylmethane, 2,4-dimethyldibenzoylmethane, 2, 5-dimethyldibenzoylmethane, 4,4'-diisopropyldibenzoylmethane, 4-methoxy-4'-tert-butyldibenzoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoylmethane, and 2,6-dimethyl-4-tert-butyl-4'-methoxydibemzoylmethane.

Patent Document 3 describes that an endermic liniment that has superior ultraviolet absorption in the UV-A region and improves stability of the ultraviolet absorption effect while preventing the discoloration of 2-ethylhexyl p-methoxycinnamate has been developed by adding a benzotriazole derivative represented by the general formula (I) to an endermic liniment containing 2-ethylhexyl p-methoxycinnamate as an ultraviolet absorbent in the UV-B.

Patent Citation 1: Japanese Patent Laid-Open H10-265357 bulletin
Patent Citation 2: Japanese Patent Laid-Open H9-2929 bulletin
Patent Citation 3: Japanese Patent Laid-Open 2005-206473 bulletin

### DISCLOSURE OF INVENTION

### TECHNICAL PROBLEM

### [First invention]

The inventors conducted earnest research on the characteristics of various ultraviolet absorbents and as a result discovered that, although the addition of a benzotriazole derivative of a specific structure and metal oxide powder to a sunscreen cosmetic causes the problem of staining due to secondary adhesion on clothing, this staining can be significantly alleviated by treating the surface of the metal oxide powder with an alkylalkoxy silane, thus completing the present invention.

The object of the present invention is to reduce the staining of clothing due to a sunscreen cosmetic containing a benzotriazole derivative of a specific structure and metal oxide powder.

### [Second invention]

The inventors conducted earnest research on ultraviolet absorbents that effectively absorb the UV-A region and as a result discovered that, although the addition of an ultraviolet absorbent having absorption in the UV-A region and metal oxide powder to a sunscreen cosmetic causes the problem of staining due to secondary adhesion on clothing, this staining can be significantly alleviated by treating the surface of the metal oxide powder with an alkylalkoxy silane, thus completing the present invention.

The object of the present invention is to reduce the staining of clothing due to a sunscreen cosmetic containing an ultraviolet absorbent in the UV-A region and inorganic powder.

### TECHNICAL SOLUTION

### [First invention: Claims 1-5]

That is, the present invention provides a sunscreen cosmetic comprising a benzotriazole derivative represented by the following general formula (I) and metal oxide powder whose surface is treated with an alkylalkoxy silane.

### General formula (I)

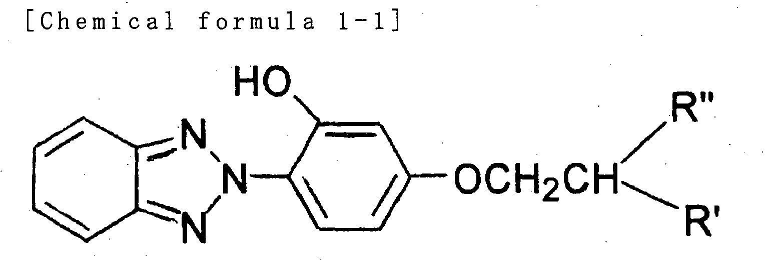

(In this formula, R' = a straight chain alkyl group of C1-C6 and R" = a straight chain alkyl group of C1-C3.)

Also, the present invention provides the aforementioned sunscreen cosmetic wherein said benzotriazole derivative is one, two or more elected from a group consisting of 2-[4-(2-ethylhexyloxy)-2-hydroxyphenyl]-2H-benzotriazole and 2-(2-hydroxy-4-isobuthoxyphenyl)-2H-benzotriazole.

Furthermore, the present invention provides the aforementioned sunscreen cosmetic wherein said alkylalkoxy silane is one, two, or more selected from a group consisting of octyltriethoxysilane and octyltrimethoxysilane.

Also, the present invention provides the aforementioned sunscreen cosmetic wherein said metal oxide powder is one, two or more selected from a group consisting of titanium oxide, zinc oxide, iron oxide, and cerium oxide.

Further, the present invention provides the aforementioned sunscreen cosmetic wherein the blend ratio of said metal oxide powder is 1-50 wt% of the total amount of the sunscreen cosmetic.

### [Second invention: Claims 6-10]

That is, the present invention provides a sunscreen cosmetic comprising an ultraviolet absorbent having absorption in the UV-A region and metal oxide powder whose surface is treated with an alkylalkoxy silane.

Also, the present invention provides the aforementioned sunscreen cosmetic wherein said ultraviolet absorbent having absorption in the UV-A region is one, two, or more selected from a group consisting of 2,4-bis {[4-(2-ethylhexyloxy)-2-hydroxy]-phenyl}-6-(4-methoxyphenyl)-1,3,5-triazine, ethylhexyl 2-cyano-3,3-diphenylacrylate, hexyl diethylaminohydroxybenzoylbenzoate, phenylene-1,4-bis (2-benzimidazyl)-3,3'-5,5'-tetrasulfonic acid and salts thereof.

Furthermore, the present invention provides the aforementioned sunscreen cosmetic wherein said alkylalkoxy silane is one, two, or more selected from a group consisting of octyltriethoxysilane and octyltrimethoxysilane.

Also, the present invention provides the aforementioned sunscreen cosmetic wherein said metal oxide powder is one, two, or more selected from a group consisting of titanium oxide, zinc oxide, iron oxide, and cerium oxide.

Furthermore, the present invention provides the aforementioned sunscreen cosmetic wherein the blend ratio of said metal oxide powder is 1-50 wt% of the total amount of the sunscreen cosmetic.

### ADVANTAGEOUS EFFECTS

### [First and second inventions]

(1) The sunscreen cosmetic of the present invention reduces the staining of clothing. Therefore, the ultraviolet absorbent and inorganic powder can be added to a sunscreen cosmetic at a high blend ratio.
(2) The sunscreen cosmetic of the present invention has a superior ultraviolet absorbing effect in the UV-A region and the inorganic powder gives it a superior ultraviolet shielding effect.

### BRIEF DESCRIPTION OF DRAWINGS

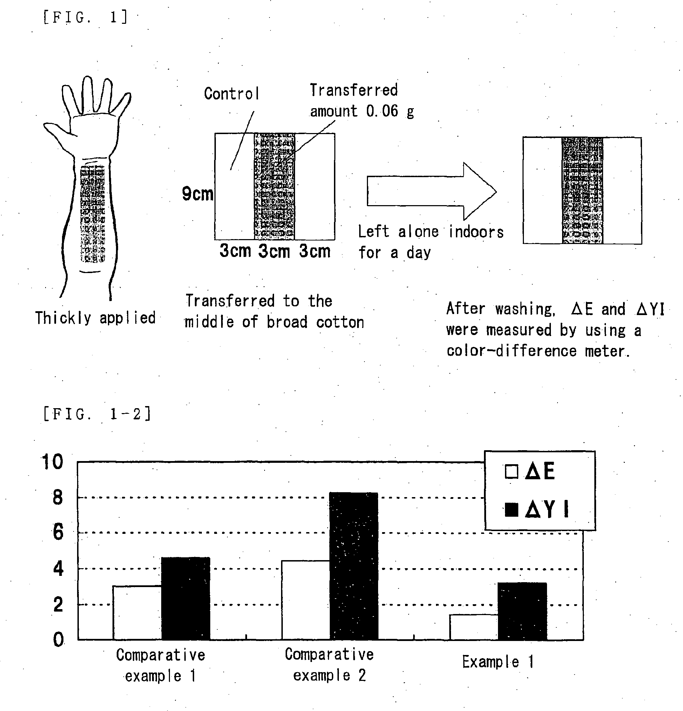

[FIG. 1] FIG. 1 illustrates how to measure the staining tendency.
[FIG. 1-2]FIG. 1-2 shows the measurement results of the staining tendency.
[FIG. 2-2]FIG. 2-2 shows the measurement results of the staining tendency.

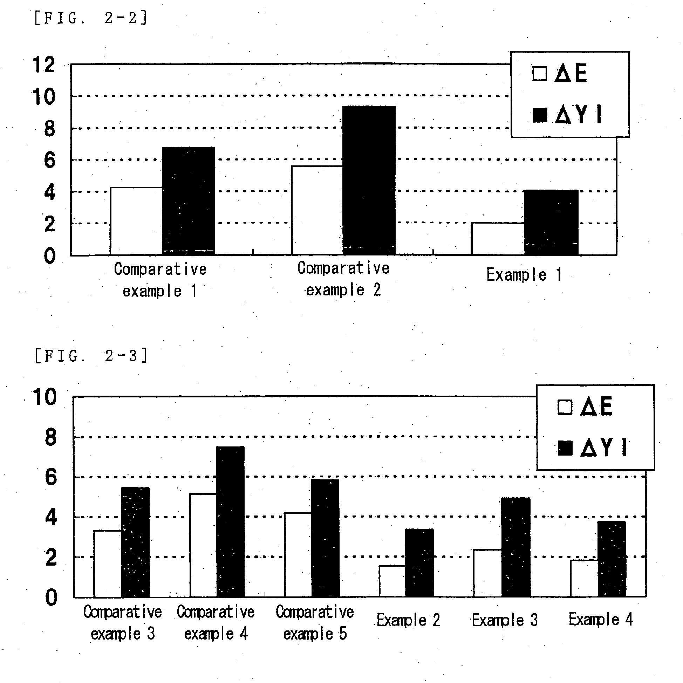

[FIG. 2-3]FIG. 2-3 shows the measurement results of the staining tendency.

### BEST MODE FOR CARRYING OUT THE INVENTION

The present invention is described in detail below.

### [First invention]

### "Benzotriazole derivative represented by general formula (I)"

The benzotriazole derivative of general formula (I) is a prior art chemical compound, which is synthesized in the following manner. A common method is to use sodium nitrite and such to turn o-nitroaniline into a diazonium salt and then couple this with phenol to synthesize a monoazo compound, and reduce it to obtain benzotriazole.

### (Method A)

First process 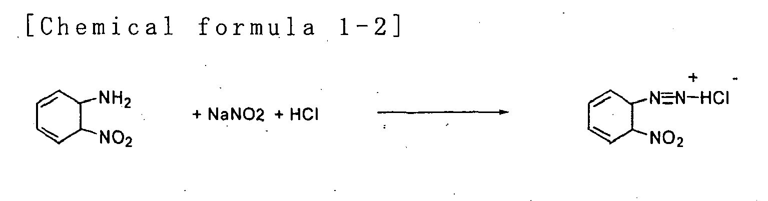
Second process 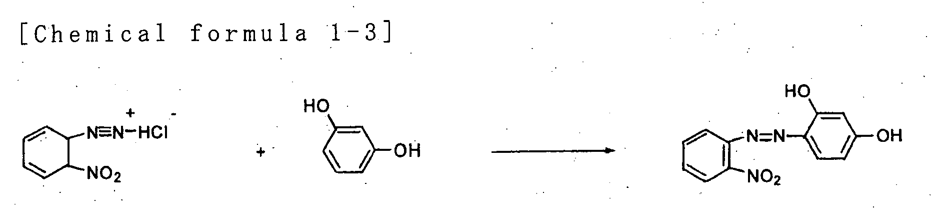
Third process 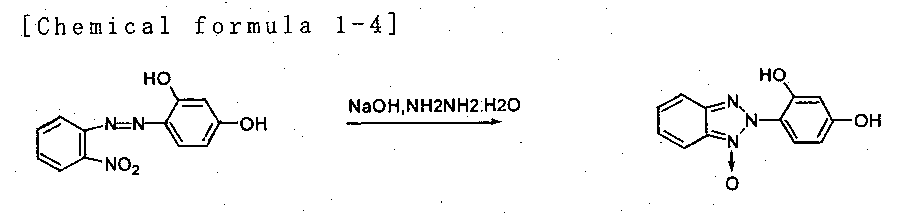
Fourth process 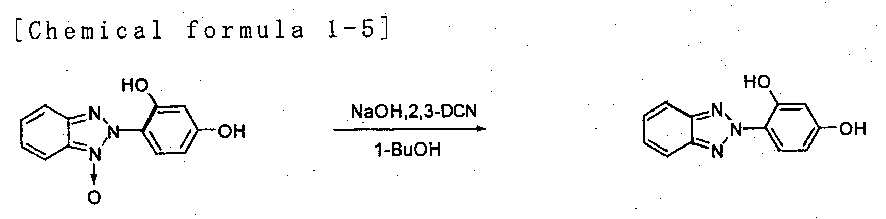 (In this formula, 2,3-DCN denotes 2,3-dichloro-1,4-naphthoquinone.)
Fifth process 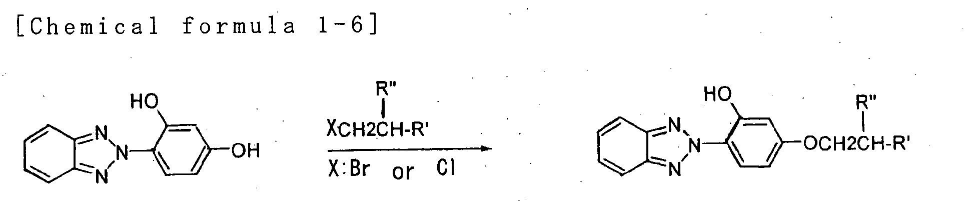 (In this formula, R = H or CH3, R' = a straight chain alkyl group of C1-C6 and R" = a straight chain alkyl group of C1-C3.)

### (Method B)

First process 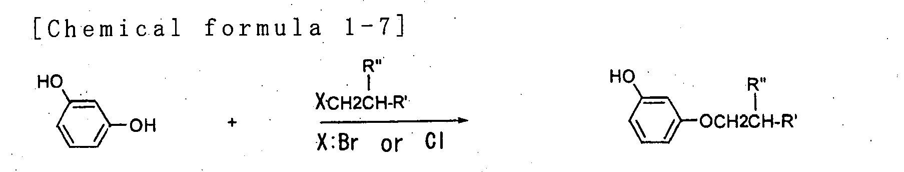
Second process 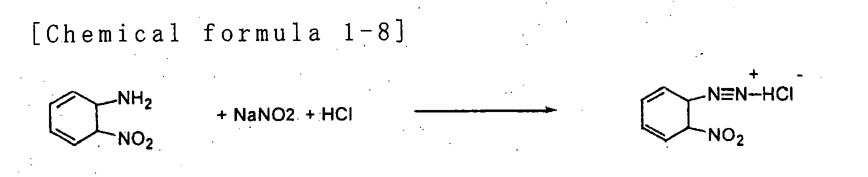
Third process 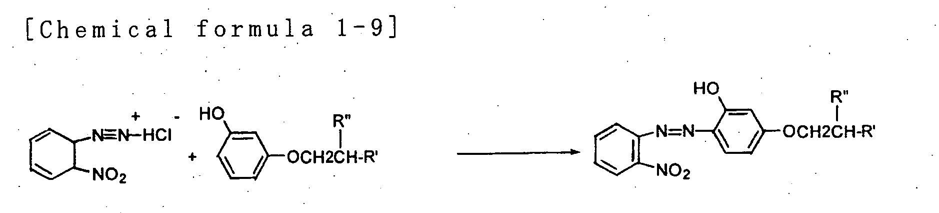
Fourth process 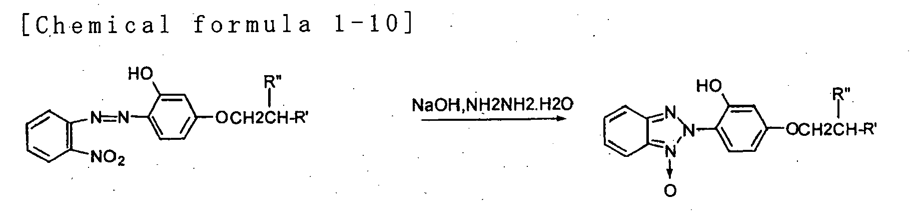
Fifth process 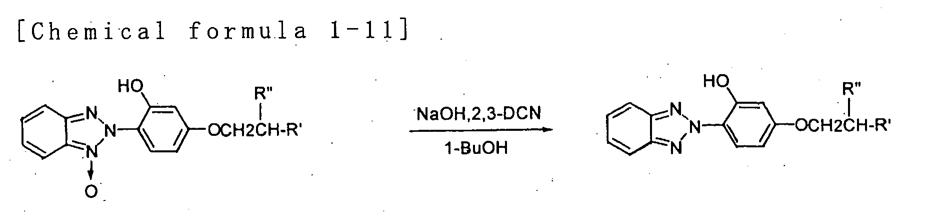 (In this formula, R' = a straight chain alkyl group of C1-C6 and R" = a straight chain alkyl group of C1-C3. 2,3-DCN denotes 2,3-dichloro-1,4-naphthoquinone.)

Refluxing a corresponding benzotriazole and alkylated halogen in a mixed solvent of methylisobutylketone and dimethylformamide produces the compound of general formula (I) with a particularly high yield.
Specifically, 6-(2H-bonzotriazole-2-yl) resorcinol is put into a four-neck flask equipped with a thermometer and refluxing cooler, to which methylisobutylketone and dimethylformamide are added and the mixture is stirred. To this, sodium carbonate and 2-ethylhexyl bromide are added and the temperature is raised to the refluxing temperature while stirring. After the mixture is stirred while the refluxing temperature is maintained for a prescribed amount of time, methylisobutylketone is recovered under normal pressure; the remaining oil is then rinsed with water to remove excess sodium carbonate and inorganic byproducts to obtain liquid 2-[4-(2-ethylhexyloxy)-2-hydroxyphenyl]-2H-benzotriazole at a high yield.

The blend ratio of the benzotriazole derivative represented by general formula (I) is selected as appropriate for the target product; it is preferably 0.5-10 wt%, more preferably 1-5 wt%, of the total amount of the sunscreen cosmetic.

### [Second invention]

### "Ultraviolet absorbent having absorption in the UV-A region"

In the present invention "an ultraviolet absorbent having absorption in the UV-A region" refers to what is usually blended into cosmetics as an ultraviolet absorbent in the.UV-A region. A preferable example is one, two, or more types of ultraviolet absorbent selected from a group consisting of 2,4-bis{[4-(2-ethylhexyloxy)-2-hydroxy]phenyl}-6-(4-methoxyphenyl)-1,3,5-triazine, ethylhexyl 2-cyano-3,3-diphenylacrylate, hexyl diethylaminohydroxybenzoylbenzoate, phenylene-1,4-bis (2-benzimidazyl)-3,3'-5,5'-tetrasulfonic acid and salts thereof. Their structural formulas are shown below. Commercial products can be used preferably for these. "2,4-bis{[4-(2-ethylhexyloxy)-2-hydroxy]-phenyl}-6-(4-methoxyphenyl)-1,3,5,-triazine" 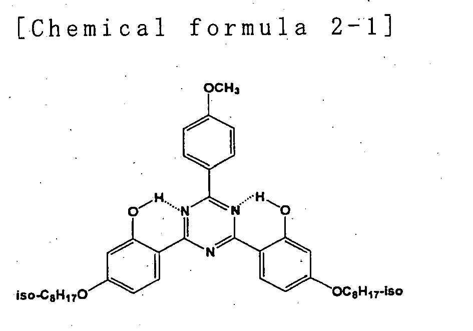 "Ethylhexyl 2-cyano-3,3-diphenylacrylate" 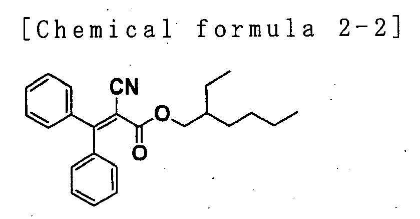 "Hexyl diethylaminohydroxybenzoylbenzoate" 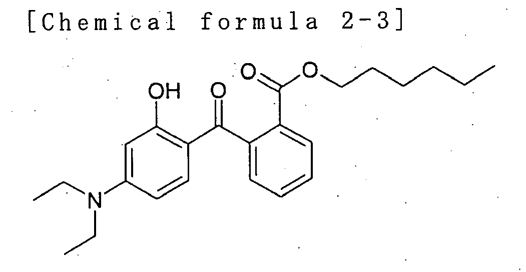 "Phenylene-1,4-bis (2-benzimidazyl)-3,3'-5,5'-tetrasulfonic acid bis-sodium salt" (When phenylene-1,4-bis (2-benzimidazyl)-3,3'-5,5'-tetrasulfonic acid and caustic soda are blended into a sunscreen cosmetic, a sodium salt having the following structure is obtained.)

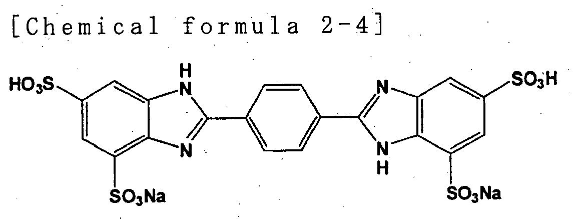

The blend ratio of the aforementioned ultraviolet absorbent is selected as appropriate for the target product; it is preferably 0.5-15 wt%, more preferably 1-3 wt%, of the total amount of the sunscreen cosmetic. The significance of the present invention is particularly great when the blend ratio is high, i.e. 2 wt% or more.

### [First and second inventions]

### "Metal oxide powder whose surface is treated with an alkylalkoxy silane"

Selection of the metal oxide powder used in the present invention is not limited in particular as long as it causes staining of clothing when used together with the aforementioned ultraviolet absorbent; preferable examples include titanium oxide, zinc oxide, and cerium oxide. Particularly preferable are fine particle powders of titanium oxide or zinc oxide. Furthermore, use of a fine particle powder having an average particle size of 0.15 µ m or less is preferable because then, when the cosmetic is applied, a natural transparent finish without a powdery look can be achieved.
One, two, or more types of metal oxide powder are treated with alkylalkoxy silane on the surface and blended into a sunscreen cosmetic.

The alkylalkoxy silane that functions as a staining prevention agent in the present invention is preferably octyltriethoxysilane or octyltrimethoxysilane. The method of the surface treatment is not limited in particular; examples include the dry treatment in which, for example, a high speed stirrer such as a henschel mixer and super mixer is used to mix the alkylalkoxy silane and the metal oxide powder and the wet treatment
in which, for example, the alkylalkoxy silane is added to a slurry of the metal oxide powder, followed by stirring and mixing.

The coating amount of the alkylalkoxy silane is preferably 1-10 wt%, more preferably 3-8 wt%, of the metal oxide powder. If it is less than 1 wt%, then the effect of the present invention is not manifested efficiently; if it is more than 10 wt%, then the ultraviolet protection effect per unit amount of the metal oxide may decrease.

The blend ratio of the metal oxide powder whose surface is treated with alkylalkoxy silane is not limited and is selected as appropriate for the target product; it is preferably 1-50 wt%, more preferably 5-30 wt%, and even more preferably 10-20 wt%, of the total amount of the sunscreen cosmetic. If it is more than 50 wt%, then a squeaky sensation occurs at the time of application and the color may become white, losing the natural look. If it is less than 1 wt%, then an effective staining prevention effect cannot be expected.

In addition to the aforementioned essential ingredients, other ingredients commonly used in cosmetics can be blended in as necessary in the sunscreen cosmetic of the present invention; examples of such ingredients include whitening agents, humectants, antioxidants, oil-based ingredients, other ultraviolet absorbents, surfactants, thickeners, alcohols, powder ingredients, coloring agents, water-based ingredients, water, and various skin nutrients, and the sunscreen cosmetic can be prepared with a conventional method. Specific examples include the following ingredients:

Oil components such as avocado oil, macadamia nut oil, corn oil, olive oil, rapeseed oil, evening primrose oil, castor oil, sunflower oil, tea seed oil, rice bran oil, jojoba oil, cacao oil, coconut oil, squalene, beef tallow, Japanese core wax, beeswax, candelilla wax, carnauba wax, whale wax, lanolin, liquid paraffin, polyoxyethylene (8 mole) oleyl alcohol ether, glyceryl monooleate, cyclomethicone, dimethylpolysiloxane, and diphenylpolysiloxane.
Higher alcohols such as caprylic alcohol, lauryl alcohol, myristyl alcohol, cetyl alcohol, cholesterol, and phytosterol.
Higher fatty acids such as caprylic acid, lauric acid, myristic acid, palmitic acid, stearic acid, behenic acid, lanolin fatty acid, linoleic acid, and linolenic acid.
Humectants such as polyethylene glycol, glycerin, sorbitol, xylitol, maltitol, mucopolysaccharide, hyaluronic acid, chondroitin sulfate, and chitosan.
Thickeners such as methyl cellulose, ethyl cellulose, Arabic gum, and polyvinyl alcohol.
Organic solvents such as ethanol and 1,3-butylene glycol.
Antioxidants such as butylhydroxytoluene, tocopherol, and phytic acid.
Antibacterial preservatives such as benzoic acid, salicylic acid, sorbic acid, paraoxybenzoic esters (ethylparaben and butylparaben, for example), and hexachlorophene.
Amino acids such as glycine, alanine, valine, leucine, serine, threonine, phenyalanine, tyrosine, aspartic acid, asparagine, glutamine, taurine, arginine, and histidine, as well as hydrochlorides thereof.
Organic acids such as acyl sarcosinic acid (sodium lauroyl sarcosinate, for example), glutathione, citric acid, malic acid, tartaric acid, and lactic acid.
Vitamins such as vitamin A and its derivatives, vitamin B's including vitamin B6 hydrochloride, vitamin B6 tripalmitate, vitamin B6 dioctanoate, vitamin B2 and its derivatives, vitamin B12, and vitamin B15 and its derivatives, vitamin C's including ascorbic acid, ascorbic malic esters (salts), and ascorbic dipalmitate, vitamin E' s including α -tocopherol, β -tocopherol, γ -tocopherol, vitamin E acetate, and vitamin E nicotinate, vitamin D's, vitamin H, pantothenic acid, and pantethine.
Various drugs such as nicotinamide, benzyl nicotinate, γ-oryzanol, allantoin, glycyrrhizic acid (salt), glycyrrhizic acid and its derivatives, hinokitiol, musidine, bisabolol, eucalyptol, thymol, inositol, saponins (saikosaponin, carrot saponin, gourd saponin, soapberry saponin, etc.), pantothenylethyl ether, ethynylestradiol, tranexamic acid, cepharanthine, and placenta extract.
Natural extracts from Rumex japonicus, Sophora flavescens, Nuphar japonica, orange, sage, thyme, yarrow, mallow, smilax, swertia, Ligusticum acutilobum, bitter orange peel, birch, horsetail, gourd, horse chestnut, creeping saxifrage, arnica, lily, mugwort, Paeonia lactiflora, aloe, gardenia, Chamaecyparis pisifera, etc. extracted by using an organic solvent, alcohol, polyhydric alcohol, water, hydroalcohol, etc.
Cation surfactants such as stearyltrimethylammonium chloride, benzalkonium chloride, and lauryl amine oxide.
Sequestering agents such as disodium edetate, trisodium edetate, sodium citrate, sodium polyphosphate, sodium metaphosphate, and gluconic acid.
Perfumes, scrubbing agents, purified water, etc.

Particularly preferable base agents for the sunscreen cosmetic of the present invention are oil components including decamethylcyclopentasiloxane, isononyl isononanoate, dimethylpolysiloxane, heptamethyloctyltrisiloxane, trimethylsiloxysilicic acid, liquid paraffin, squalane, cetyl isooctanoate, triglyceride isooctanoate, and di-2-ethylhexyl succinate. The present invention is used preferably in sunscreen cosmetics that use decamethylcyclopentasiloxane as the main base agent.

The sunscreen cosmetic of the present invention can be used in any product form such as ointments, cream, emulsion, and lotions. The dosage form is not limited either.

### EXAMPLES

The invention is described in specific detail through Examples. The present invention is not limited to these Examples.

### [First invention]

### <Synthesis example of the benzotriazole derivative represented by general formula (I)>

### "Synthesis example 1-1: Synthesis of 2-[4-(2-ethylhexyloxy)-2-hydroxyphenyl]-2H-benzotriazole "

45.4 g (0.20 moles) of 6-(2H-benzotriazole-2-yl) resorcinol, synthesized by using a conventional method, was put into a 500 ml four neck flask equipped with a thermometer and a reflux cooling apparatus, to which 50 ml of methylisobutylketone and 4.0 g of dimethylformamide were added, followed by stirring. Into this, 25.4 g (0.24 moles) of sodium carbonate and 77.2 g (0.40 moles) of 2-ethylhexyl bromide were added and the mixture was heated up to the refluxing temperature while being stirred. After stirring the mixture for 15 hours while maintaining the refluxing temperature, methylisobutylketone was recovered under normal pressure and the remaining oil was then rinsed with water to remove excess sodium carbonate and inorganic byproducts. This oil was distilled under a reduced pressure to obtain 52.1 g of a 220-225°C/0.2-0.3 mmHg fraction, which was yellow and transparent. This compound was liquid at ordinary temperatures; the yield was 76.7% and the HPLG purity was 99.0%.

### "Synthesis example 1-2: Synthesis of 2-[2-hydroxy-4-isobutoxyphenyl]-2H-benzotriazole"

Instead of 2-ethylhexyl bromide, the equal number of moles of isobutyl bromide was used in the same manner as in Synthesis example 1. Slightly yellow-gray-white powdery crystals were obtained at a yield of 72.5%. The m.p. was 120.0-120.8°C, λ max = 345.6 nm, and ε = 21750.

### "Metal oxide powder whose surface is treated with alkylalkoxy silane"

2 k g of titanium oxide powder (average particle size 0.015 x 0.06 µ m) was turned into a water based slurry; 20% sulfuric acid was used to bring the pH to 3; 100 g of octyltriethoxysilane was added; after 30 minutes of stirring, sodium hydroxide was used to bring the pH to 10, followed by one hour of maturation. This was then neutralized, filtered, rinsed, dried, and then crushed by using a jet mill to obtain titanium oxide powder whose surface is treated with octyltriethoxysilane.
The titanium oxide powder can be replaced with powder of zinc oxide, iron oxide, or cerium oxide to obtain powder of zinc oxide, iron oxide, or cerium oxide whose surface is treated with octyltriethoxysilane.
In the same manner, each metal oxide powder whose surface is treated with octyltrimethoxysilane can be obtained.

W/0 sunscreens shown in Table 1-1 were prepared using a conventional method and the staining tendency due to secondary adhesion was investigated.

**[Table 1-1]**

| | Comparative example 1 | Comparative example 2 | Example 1 |
|---|---|---|---|
| Decanethylcyclopentasiloxane | 21.0 | 21.0 | 21.0 |
| Dimethylpolysiloxane | 2.0 | 2.0 | 2.0 |
| (Vinyl Dimethicone/Methiconesilsequioxane) Crosspolymer | 5.0 | 5.0 | 5.0 |
| Lauryl PEG-9 polydimethylsiloxyethyl dimethicone | 1.5 | 1.5 | 1.5 |
| Trimethylsiloxysilicic acid | 5.0 | 5.0 | 5.0 |
| Triglyceride isooctanoate | 5.0 | 5.0 | 5.0 |
| Cetyl isooctanoate | 5.0 | 5.0 | 5.0 |
| Isononyl isononanoate | 5.0 | 5.0 | 5.0 |
| 2-ethylhexyl paramethoxycinnamate | 7.5 | 7.5 | 7.5 |
| Benzotriazole derivative of Synthetic example 1 | 8.0 | 8.0 | 8.0 |
| Stearic acid-treated titanium oxide | 0.0 | 12.0 | 0.0 |
| Octyltriethoxysilane-treated titanium oxide | 0.0 | 0.0 | 12.0 |
| Dimethyldistearyl ammonium hectorite | 0.2 | 0.2 | 0.2 |
| Purified water | 24.45 | 12.45 | 12.45 |
| Trisodium edetate | 0.05 | 0.05 | 0.05 |
| Dipropylene glycol | 5.0 | 5.0 | 5.0 |
| Glycer in | 2.0 | 2.0 | 2.0 |
| Ethanol | 3.0 | 3.0 | 3.0 |
| Phenoxy ethanol | 0.3 | 0.3 | 0.3 |
| Total | 100.0 | 100.0 | 100.0 |

### "Method for measuring the staining tendency"

As shown in FIG. 1, the sample was applied thickly on an arm and then transferred to the middle of broad cotton (transferred amount approximately 0.06 g); after being left alone indoors for a day, it was washed using a conventional laundry detergent and Δ E and Δ YI were measured by using a spectrophotometer (CM-2002 from Minolta, currently Konica Minolta Sensing Co., Ltd.). The results are shown in Figure 1-2.

FIG. 1-2 indicates that the staining tendency was reduced significantly for Example 1, compared with Comparative example 1, in which the metal oxide powder was not blended in, and Comparative example 2, in which a hydrophobicizing agent other than alkylalkoxy silane was used for the treatment.

The following are formulation examples of the sunscreen cosmetic of the present invention. Each of these examples is a sunscreen cosmetic that has a reduced staining tendency on clothing and exhibits superior ultraviolet absorption capacity in the UV-A region.

### "Example 1-2: Sunscreen cosmetic W/0 emulsion"

| | wt% |
|---|---|
| 1. Dimethylpolysiloxane | 1 |
| 2. Decamethylcyclopentasiloxane | 25 |
| 3. Trimethylsiloxysilicic acid | 5 |
| 4. Lauryl PEG-9 polydimethylsiloxyethyl dimethicone | 1 |
| 5. Isononyl isononoate | 5 |
| 6. Butylethylpropanediol | 0.5 |
| 7. 2-ethylhexyl paramethoxycinnamate | 7.5 |
| 8. Benzotriazole derivative of Synthesis example 2 | 5 |
| 9. Ethylhexyl 2-cyano-3,3-diphenylacrylate | 5 |
| 10. Dimethyldistearylammonium hectorite | 0.5 |
| 11. Spherical polyalkyl acrylate powder | 5 |
| 12. Zinc oxide powder whose surface is treated with octyltriethoxysilane | 15 |
| 13. Dipropylene glycol | 5 |
| 14. Phenoxy ethanol | 0.5 |
| 15. Ethanol | 2 |
| 16. Purified water | Balance |

### Preparation method

The water phase was gradually added to the oil phase; after the addition a stirrer was used to homogenize the emulsified particles to complete the preparation.

### "Example 1-3: Sunscreen cosmetic 0/W emulsion"

| | wt% |
|---|---|
| 1. Polyoxyethylene hydrogenated castor oil | 1 |
| 2. Dimethicone copolyol | 0.5 |
| 3. Decamethylcyclopentasiloxane | 15 |
| 4. Isostearic acid | 0.5 |
| 5. Phenyl trimethicone | 1 |
| 6. Titanium oxide powder whose surface is treated with octyltrimethoxysilane | 5 |
| 7. Benzotriazole derivative of Synthesis example 1 | 5 |
| 8. 2-ethylhexyl-paramethoxycinnamate | 5 |
| 9. Citric acid | 0.01 |
| 10. Sodium citrate | 0.09 |
| 11. Phenoxy ethanol | 0.5 |
| 12. Ethanol | 5 |
| 13. Dynamite glycerin | 1 |
| 14. Succinoglucan | 0.2 |
| 15. Cellulose gum | 1 |
| 16. Ion-exchanged water | Balance |

### Preparation method

After preparing the water phase, 9-16, it was gradually added to the oil phase, 1-8, followed by stirring by means of a homomixer.

### "Example 1-4: Self tanning cosmetic"

| Part A | wt% |
|---|---|
| 1. 1,3-butylene glycol | 5 |
| 2. Glycerin | 2 |
| 3. Dihydroxyacetone | 5 |
| 4. Disodium edetate | 0.05 |
| 5. Paraben | Appropriate amount |
| 6. Ion-exchanged water | Balance |

| Part B | |
|---|---|
| 7. Glyceryl stearate | 4 |
| 8. Behenyl alcohol | 3 |
| 9. Stearyl alcohol | 2 |
| 10. Silicone oil | 6 |
| 11. Hydrogenated palm oil | 2 |
| 12. Liquid paraffin | 2 |
| 13. Zinc oxide powder whose surface is treated with octyltrimethoxysilane | 3 |
| 14. Benzotriazole derivative of Synthesis example 2 | 2 |
| 15. Ethylhexyl 2-cyano-3,3-diphenylacrylate | 5 |
| 16. Perfume | Appropriate amount |

### Preparation method

Disodium edetate, dihydroacetone, glycerin, and Paraben heated and dissolved in 1,3-butylene glycol were added to the ion-exchanged water of part A. Each ingredient of part B was thoroughly dissolved and heated, and then added to part A, followed by emulsification. This was cooled to obtain the self tanning cream.

### [Second invention]

### "Metal oxide powder whose surface is treated with alkylalkoxy silane"

2 kg of titanium oxide powder (average particle size 0.015 x 0.06 µm) was turned into a water based slurry; 20% sulfuric acid was used to bring the pH to 3; 100 g of octyltriethoxysilane was added; after 30 minutes of stirring, sodium hydroxide was used to bring the pH to 10, followed by one hour of maturation. This was then neutralized, filtered, rinsed, dried, and then crushed by using a jet mill to obtain titanium oxide powder whose surface is treated with octyltriethoxysilane.
The titanium oxide powder can be replaced with powder of zinc oxide, iron oxide, or cerium oxide to obtain powder of zinc oxide, iron oxide, or cerium oxide whose surface is treated with octyltriethoxysilane.
In the same manner, each metal oxide powder whose surface is treated with octyltrimethoxysilane.

W/0 sunscreens shown in Tables 2-1 and 2-2 were prepared using a conventional method and the staining tendency due to secondary adhesion was investigated.

**[Table 2-1]**

| | Comparative example 1 | Comparative example 2 | Example 1 |
|---|---|---|---|
| Decamethylcyclopentasiloxane | 21. 0 | 21.0 | 21.0 |
| Dimethylpolysiloxane | 2.0 | 2.0 | 2.0 |
| (Vinyl Dimethicone/Methiconesilsequioxane) Crosspolymer | 5.0 | 5.0 | 5.0 |
| Lauryl PEG-9 polydimethylsiloxyethyl dimethicone | 1.5 | 1.5 | 1.5 |
| Trimethylsiloxysilicic acid | 5.0 | 5.0 | 5.0 |
| Triglyceride isooctanoate | 5.0 | 5.0 | 5.0 |
| Cetyl isooctanoate | 5.0 | 5.0 | 5.0 |
| Isononyl isononanoate | 5.0 | 5.0 | 5.0 |
| 2-ethylhexyl paramethoxycinnamate | 7.5 | 7.5 | 7.5 |
| 2,4-bis {[4-(2-ethylhexyloxy)-2-hydroxy] phenyl}-6-(4-inethoxyphenyl)-1,3,5-triazine | 2.0 | 2.0 | 2.0 |
| Stearic acid-treated titanium oxide | 0.0 | 12.0 | 0.0 |
| Octyltriethoxysilane-treated titanium oxide | 0.0 | 0.0 | 12.0 |
| Dimethyldistearyl ammonium hectorite | 0.2 | 0.2 | 0.2 |
| Purified water | 30.45 | 18.45 | 18.45 |
| Trisodium edetate | 0.05 | 0.05 | 0.05 |
| Dipropylene glycol | 5.0 | 5.0 | 5.0 |
| Glycerin | 2.0 | 2.0 | 2.0 |
| Ethanol | 3.0 | 3.0 | 3.0 |
| Phenoxy ethanol | 0.3 | 0.3 | 0.3 |
| Total | 100.0 | 100.0 | 100.0 |

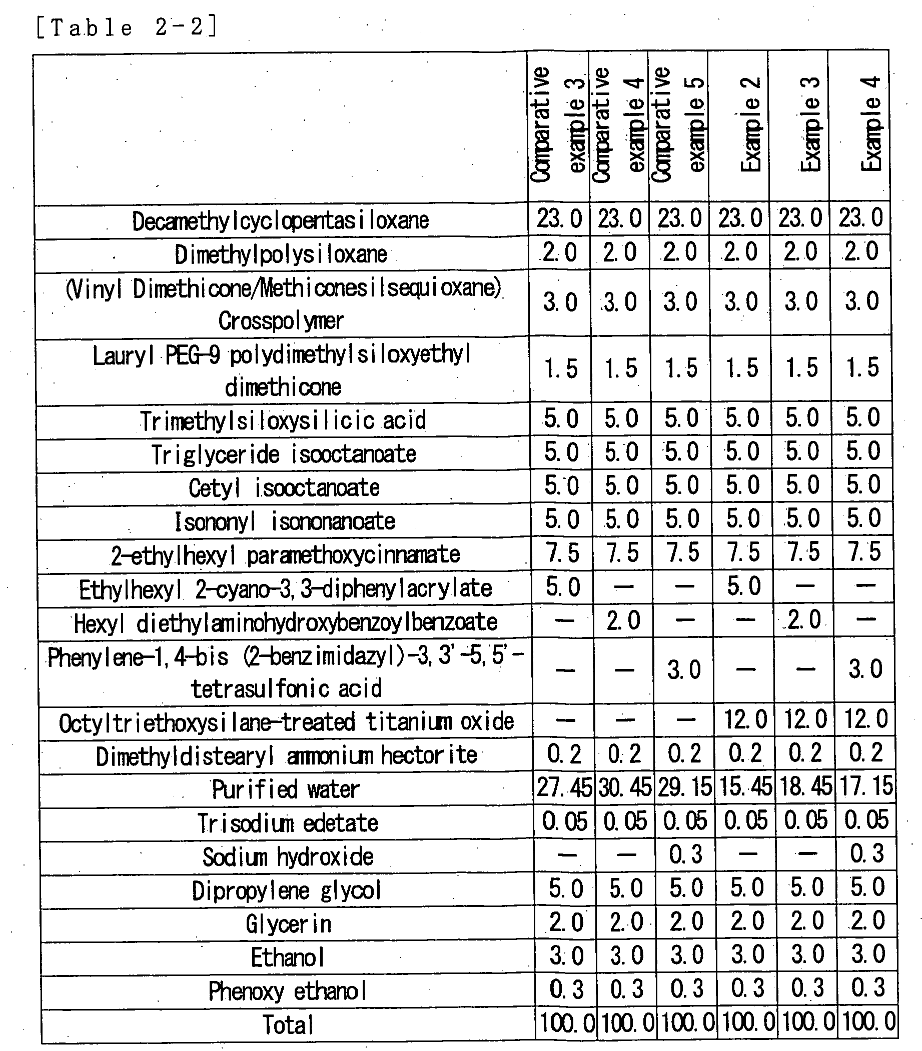

### "Method for measuring the staining tendency"

As shown in FIG. 1, the sample was applied thickly on an arm and then transferred to the middle of broad cotton (transferred amount approximately 0.06 g); after being left alone indoors for a day, it was washed using a conventional laundry detergent and Δ E and Δ YI were measured by using a spectrophotometer (CM-2002 from Minolta, currently Konica Minolta Sensing Co., Ltd.). The results are shown in FIG. 2-2 and FIG. 2-3.

FIG. 2-2 and FIG. 2-3 indicate that the staining tendency was reduced significantly for Examples 1-4, compared with Comparative example 1,
in which the metal oxide powder was not blended in, and Comparative examples 2-5, in which a hydrophobicizing agent other than alkylalkoxy silane was used for the treatment.

The following are formulation examples of the sunscreen cosmetic of the present invention. Each of these examples is a sunscreen cosmetic that has a reduced staining tendency on clothing and exhibits superior ultraviolet absorption capacity in the UV-A region.

### "Example 2-5: Sunscreen cosmetic W/0 emulsion"

| | wt% |
|---|---|
| 1. Dimethylpolysiloxane | 1 |
| 2. Decamethylcyclopentasiloxane | 25 |
| 3. Trimethylsiloxysilicic acid | 5 |
| 4. Lauryl PEG-9 polydimethylsiloxyethyl dimethicone | 1 |
| 5. Isononyl isononoate | 5 |
| 6. 2-ethylhexyl paramethoxycinnamate | 7.5 |
| 7. Butylethylpropanediol | 0.5 |
| 8. Hexyl diethylaminohydroxybenzoylbenzoate | 2 |
| 9. Ethylhexyl 2-cyano-3,3-diphenylacrylate | 5 |
| 10. Dimethyldistearylammonium hectorite | 0.5 |
| 11. Spherical polyalkyl acrylate powder | 5 |
| 12. Zinc oxide powder whose surface is treated with octyltriethoxysilane | 15 |
| 13. Dipropylene glycol | 5 |
| 14. Phenoxy ethanol | 0.5 |
| 15. Ethanol | 2 |
| 16. Phenylene-1,4-bis (2-benzimidazyl)-3,3'-5,5'-tetrasulfonic acid | 2 |
| 17. Triethanolamine | 1 |
| 18. Purified water | Balance |

### Preparation method

The water phase was gradually added to the oil phase; after the addition a stirrer was used to homogenize the emulsified particles to complete the preparation.

### "Example 2-6: Sunscreen cosmetic 0/W emulsion"

| | wt% |
|---|---|
| 1. Polyoxyethylene hydrogenated castor oil | 1 |
| 2. Dimethicone copolyol | 0.5 |
| 3. Decamethylcyclopentasiloxane | 15 |
| 4. Isostearic acid | 0.5 |
| 5. Phenyl trimethicone | 1 |
| 6. Titanium oxide powder whose surface is treated with octyltrimethoxysilane | 5 |
| 7. 2,4-bis{[4-(2-ethylhexyloxy)-2-hydroxy]-phenyl}-6-(-4-methoxyphenyl)-1,3,5-triazine | 2 |
| 8. Ethylhexyl 2-cyano-3,3-diphenylacrylate | 5 |
| 9. 2-ethylhexyl-paramethoxycinnamate | 5 |
| 10. Citric acid | 0.01 |
| 11. Sodium citrate | 0.09 |
| 12. Phenoxy ethanol | 0.5 |
| 13. Ethanol | 5 |
| 14. Dynamite glycerin | 1 |
| 15. Succinoglucan | 0.2 |
| 16. Cellulose gum | 1 |
| 17. Ion-exchanged water | Balance |

### Preparation method

After preparing the water phase, 10-17, it was gradually added to the oil phase, 1-9, followed by stirring by means of a homomixer.

### "Example 2-7: Self tanning cosmetic"

| Part A | wt% |
|---|---|
| 1. 1,3-butylene glycol | 5 |
| 2. Glycerin | 2 |
| 3. Dihydroxyacetone | 5 |
| 4. Disodium edetate | 0.05 |
| 5. Paraben | Appropriate amount |
| 6. Ion-exchanged water | Balance |
| Part B | |
| 7. Glyceryl stearate | 4 |
| 8. Behenyl alcohol | 3 |
| 9. Stearyl alcohol | 2 |
| 10. Silicone oil | 6 |
| 11. Hydrogenated palm oil | 2 |
| 12. Liquid paraffin | 2 |
| 13. Zinc oxide powder whose surface is treated with octyltrimethoxysilane | 3 |
| 14. Hexyl diethylaminohydroxybenzoylbenzoate | 2 |
| 15. Ethylhexyl 2-cyano-3,3-diphenylacrylate | 5 |
| 16. Perfume | Appropriate amount |

### Preparation method

Disodium edetate, dihydroacetone, glycerin, and paraben heated and dissolved in 1,3-butylene glycol were added to the ion-exchanged water of part A. Each ingredient of part B was thoroughly dissolved and heated, and then added to part A, followed by emulsification. This was cooled to obtain the self tanning cream.

### INDUSTRIAL APPLICABILITY

### [First and second inventions]

The present invention can provide a sunscreen cosmetic that has a reduced staining tendency on clothing and has superior ultraviolet absorption properties in the UV-A region.

## Claims

1. A sunscreen cosmetic comprising a benzotriazole derivative represented by the following general formula (I) and metal oxide powder whose surface is treated with an alkylalkoxy silane.
General formula (I) 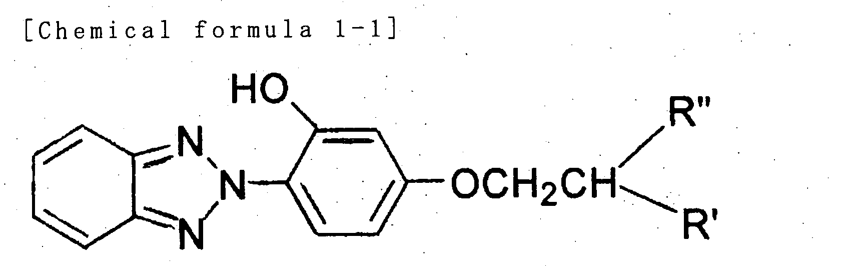 (In this formula, R' = a straight chain alkyl group of C1-C6 and R" = a straight chain alkyl group of C1-C3.)

2. The sunscreen cosmetic of claim 1 wherein said benzotriazole derivative is one, two, or more selected from a group consisting of 2-[4-(2-ethylhexyloxy)-2-hydroxyphenyl]-2H-benzotriazole and 2-(2-hydroxy-4-isobuthoxyphenyl)-2H-benzotriazole.

3. The sunscreen cosmetic of claim 1 or 2. wherein said alkylalkoxy silane is one, two, or more selected from a group consisting of octyltriethoxysilane and octyltrimethoxysilane.

4. The sunscreen cosmetic of claim 1, 2, or 3 wherein said metal oxide powder is one, two, or more selected from a group consisting of titanium oxide, zinc oxide, iron oxide, and cerium oxide.

5. The sunscreen cosmetic of claim 1, 2, 3, or 4 wherein the blend ratio of said metal oxide powder is 1-50 wt% of the total amount of the sunscreen cosmetic.

6. A sunscreen cosmetic comprising an ultraviolet absorbent having absorption in the UV-A region and metal oxide powder whose surface is treated with an alkylalkoxy silane.

7. The sunscreen cosmetic of claim 6 wherein said ultraviolet absorbent having absorption in the UV-A region is one, two, or more selected from a group consisting of 2,4-bis{[4-(2-ethylhexyloxy)-2-hydroxy]-phenyl}-6-(4-methoxyphenyl)-1,3,5-triazine, ethylhexyl 2-cyans-3,3-diphenylacrylate, hexyl diethylaminohydroxybenzoylbenzoate, phenylene-1,4-bis (2-benzimidazyl)-3, 3'-5,5'-tetrasulfonic .acid and salts thereof.

8. The sunscreen cosmetic of claim 6 or 7 wherein said alkylalkoxy silane is one, two, or more selected from a group consisting of octyltriethoxysilane and octyltrimethoxysilane.

9. The sunscreen cosmetic of claim 6, 7, or 8 wherein said metal oxide powder is one, two, or more selected from a group consisting of titanium oxide, zinc oxide, iron oxide, and cerium oxide.

10. The sunscreen cosmetic of claim 6, 7, 8, or 9 wherein the blend ratio of said metal oxide powder is 1-50 wt% of the total amount of the sunscreen cosmetic.
